# EUROPEAN PATENT APPLICATION

(11) **EP 1 159 945 A1**
(43) Date of publication of application: **05.12.2001**
(21) Application number: 01112052.4
(22) Date of filing: 25.05.2001
(51) Int. Cl.: A61F 13/62, A61F 13/56, A44B 18/00

(54) **Female component for hook and loop fastening device, method of making thereof and absorbent articles incorporating same**

(30) Priority: 30.05.2000 US 584039
(71) Applicant: First Quality Enterprises, Inc., McElhattan, Pennsylvania 17748 (US)
(72) Inventor: Karami, Hamzeh, McElhattan, PA 17748 (US)
(74) Representative: Motsch, Andreas

(57) **Abstract**

A disposable absorbent article is provided having a loop fastening material as female component of a hook and loop fastener system. The loop material comprises a web having a top surface and at least one row of filamentary yarn spanning the length of said top surface and adapted to be entangled by complementary male fastener.

## Description

### FIELD OF THE INVENTION

The present invention relates generally to hook and loop fastening system and is particularly related to the loop female fastener component of such system for use in disposable absorbent articles. This invention also relates to a method of making loop fastening materials as female component of a hook and loop fastener, and to disposable absorbent articles incorporating such female fasteners.

### BACKGROUND OF THE INVENTION

Fastening devices are essential components of disposable absorbent articles such as diapers, infant training pants, adult incontinent briefs and other similar garments. Essential for the use of these garments is that they fit tightly and comfortably around the torso of the wearer so as to prevent leakage of fluids or other bodily exudates. Accordingly, it is a common practice to provide these garments with fastening means such as adhesive tabs or mechanical fastening devices. In recent years, hook and loop fastening devices have gained widespread acceptance as the fastening devices of choice. Each hook and loop device consists of a loop fastener component defined by a material having a female surface, and a hook (male) component having a surface adapted to engage into the female loop surface in order to achieve the desired securement of the disposable article to the body of the wearer.

Loop component of hook and loop fastening devices have been described in several recent patents. For example, United States Patent No. 5,032,122 issued to Noel et al. on July 16, 1971 describes a loop fastening device having an orientable material used as a backing for the loop fastening material, also referred to as the "female fastener", "loop fastener", or "fibrous fastening material". A host of compounds are disclosed for use as the orientable backing material or the loop fastening material.

United States Patent No. 5,326,612 issued to Goulait on July 5, 1994 discloses nonwoven female component for refastenable fastening device, and describes a method of producing such female fastening component. The female component comprises at least one nonwoven web secured to a backing, and serves to admit and entangle some of the hooks of the hook and loop system to provide the desired engagement. The nonwoven web may be comprised of polyester, polyethylene or polypropylene.

Unites States Patent No. 5,569,233 issued to Goulait on October 29, 1996 describes a refastenable fastening device having multi-layer female component, and method of making the same.

Unites States Patent No. 5,647,864 issued to Allen et al. on July 15, 1997 discloses nonwoven female component for refastenable fastening device and method of making the female component. The nonwoven web used in making the female fastening component comprises carded polypropylene.

United States Patent No. 5,699,593 issued to Jackson on December 23, 1997 describes a loop fastening material for hook and loop fastener and method of making such material. The loop fastening material comprises an oriented backing sheet and a plurality of multi-filament transversely expanded yarns attached to one face of the backing sheet. The backing sheet is an oriented woven or nonwoven web or laminate.

United States Patent No. 5,858,515 issued to Stokes et al. on January 12, 1992 describes a nonwoven fabric having continuous bonded areas defining discrete unbonded areas, which is suitable for use as loop fastening material for hook and loop fastening device.

Notwithstanding the disclosure of various loop fastening materials for hook and loop devices, the use of adhesive tape/tab and/or VELCRO® remains prevalent as mechanical fasteners for disposable absorbent articles. The use of VELCRO® as fastening device is described in the aforementioned United States Patent No. 5,326,612 of Goulait, and the patents referred to therein, in column 1, lines 25-65. VELCRO® , however, is costly and adhesive tape/tabs are not as effective. While the aforementioned patents disclose loop fastening materials as alternative to VELCRO® , they are usually stiff, rigid and uncomfortable when in contact with the human skin. In addition, they are produced by complicated methods using complex apparatus and, therefore, they are expensive to produce. Accordingly, there is a need for a loop fastening material for use in hook and loop fastening device which is not only effective in its securement function but is relatively inexpensive to produce and use with disposable absorbent articles.

It is therefore an object of the present invention to provide a loop fastening material which is useful in hook and loop fastening devices.

It is another object of this invention to provide such loop fastening material using spunbond, thermal or chemically bonded nonwoven, thermoplastic films or even woven fabric.

It is a further object of this invention to provide a disposable absorbent article having a hook and loop fastening device comprising improved loop fastening material.

It is yet another object of this invention to provide a method of making such loop fastening material.

It is an additional object of this invention to incorporate such loop fastening materials in disposable absorbent articles (e.g., diapers and incontinent briefs) and to form such absorbent articles.

The foregoing and other objects of this invention will be more fully comprehended from the ensuing detailed description and the related figures in the drawings.

### SUMMARY OF THE INVENTION

In accordance with this invention, a loop fastening material is provided for use in disposable absorbent articles such as diapers, incontinent briefs and like garments. The loop fastening material serves as the female component of a hook and loop fastening device used in such absorbent articles in order to provide improved securement means therefore by relatively inexpensive and simple means.

The loop fastener material may be made by attaching filamentary yarns to one face of a single layer web of nonwoven fabric, woven fabric or a thermoplastic polymer film. Alternatively, the loop fastening material may be a laminate or composite of two webs, a lower web with the filamentary yarns attached to a surface thereof, and a second web which superimposed on said first web and is adhesively or otherwise secured thereto. The second web is perforated with spaced apertures, in one or more rows, usually corresponding to the number of rows of yarns attached to the surface of the first web. When the two webs are adhered together to form the composite or laminate, the perforated areas in the second web define a plurality of yarn sites which are visible through said perforations, which may be of various sizes and shapes, preferably circular, having a diameter of about 1/8 to about 1 inch. These yarn sites define the loop fastening material which are adapted to be entangled by complementary hooks of a male fastener such as, e.g., VELCRO® , or other male fastener element.

The female fastening material is made by a method which comprises:
(a) feeding a web of a material having a top surface, said web being selected from the group consisting of nonwoven fabric, woven fabric and a thermoplastic polymer film, said web traveling in one direction,
(b) applying adhesive to the top surface of said web as said web travels in said direction, thereby covering said top surface of the web with said adhesive,
(c) applying at least one row of filamentary yarn to said adhesive-covered surface of said web while said filamentary yarn is caused to travel in the same direction as the web, thereby producing a web with the filamentary yarn attached thereto.

The method of forming a composite female fastening material comprises
(a) feeding a first web of a material having a top surface, said web being selected from the group consisting of nonwoven fabric, woven fabric and a thermoplastic polymer film, said first web traveling in one direction,
(b) applying adhesive to the top surface of said first web as said web travels in said direction, thereby covering said top surface of the web with said adhesive,
(c) applying at least one row of filamentary yarn to said adhesive-covered surface of said web while said filamentary yarn is caused to travel in the same direction as said web, thereby producing a web with the filamentary yarn attached thereto, and
(d) feeding a second web of a material selected from the group consisting of nonwoven fabric, woven fabric, thermoplastic polymer film and gauze, said second web traveling in the same direction as the first web, said second web having at least one row of a plurality of spaced apart perforations,
whereby said second web is caused to adhere to said top surface of said first web to form a composite web with said perforations in register with the filamentary yarn on said first web thereby defining exposed sites of female fastening materials adapted to be entangled with complementary of a male fastening element.

The present invention also provides T-shaped absorbent articles, e.g., diapers, wherein the T-shaped diaper has the construction shown in commonly assigned copending application serial number 09/376,282 filed August 18, 1999, the disclosure of which is fully incorporated herein by reference. The T-shaped of the present invention, however, uses the loop fastening materials described herein in forming the diaper as will hereinafter be described in detail.

### BRIEF DESCRIPTION OF THE DRAWINGS

Figure 1 is a top view of a bonded nonwoven having several yarns attached to its surface;
Figure 2 is a side view of a typical core spun yarn which can be attached to the surface of the bonded nonwoven shown in Figure 1;
Figure 2A is a side view of another arrangement showing the yarn crocheted to the nonwoven layer with adhesive sprayed onto the nonwoven surface for improved securement of the yarn to the nonwoven layer;
Figure 2B is a side view similar to Figure 2A but wherein an additional layer of nonwoven is employed;
Figure 3 is a top, partly elevational view of a composite laminate of two webs made according to a different embodiment of the present invention, with the upper web partly peeled away to show the yarns on the unbonded areas;
Figure 4 is a sectional view taken along the line 4-4 of Figure 3;
Figure 5 is a schematic representation of the method of forming female fastener material in accordance with the present invention;
Figure 6 is a plan stretched view of a disposable diaper incorporating female loop fastener of this invention, and
Figure 7 is a plan stretched view of a diaper similar to Figure 6 but using a composite web of a female fastening material shown in Figure 3.

Like reference numerals in different figures of the drawings denote like parts.

### DETAILED DESCRIPTION OF THE INVENTION

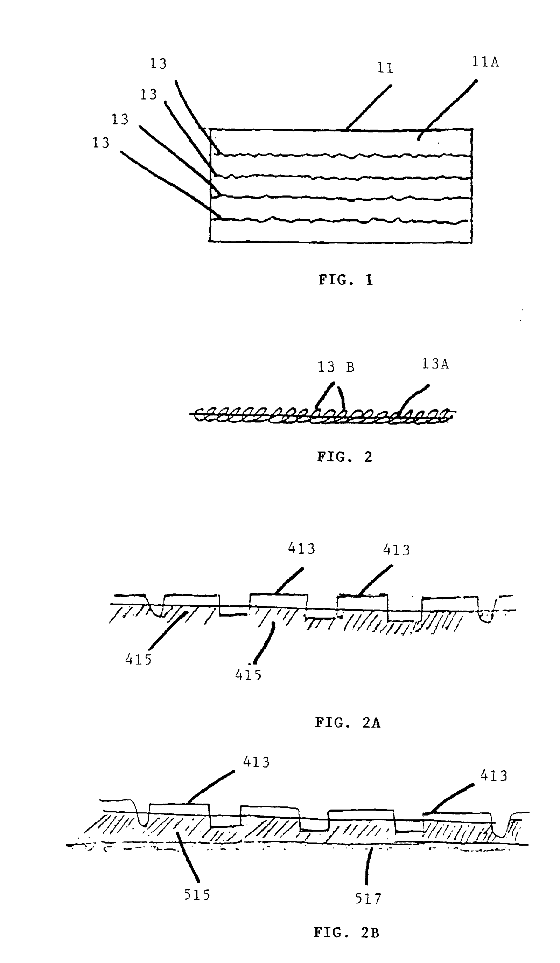

Referring to Figure 1, there is shown a web or base layer 11 which may be a bonded nonwoven fabric having a top face or surface 11A on which a plurality of filamentary yarns 13 are attached in a generally parallel, continual or continuous and spaced apart relation. The bonded nonwoven web 11 may be thermally bonded, chemically bonded or spunbond of the type generally known in the prior art. Illustrative nonwoven webs are described in the aforementioned United States Patent No. 5,858,515 which also describes various methods of producing spunbond nonwoven from melt-spun filaments.

The yarns 13 which are attached to the face or surface 11A of the nonwoven web may be core spun yarns, air covered yarns or other yarns known in the art. A variety of suitable yarns are described in the aforementioned United States Patent No. 5,699,593 and are readily available commercially. Suitable yarns include twisted yarns which generally consist of a core 13A made of Lycra® , available from E.I. du Pont de Nemour Company, with fibers 13B spun or twisted around the core 13A, as shown in Figure 2. The yarns 13 are desirably multi-filament yarns with the individual filaments being formed from a variety of known filament-forming materials such as polyolefin (e.g., polyethylene and polypropylene), polyester, polyamide and other known filament-forming materials.

The yarns 13 are adhesively attached to the surface 11A of the nonwoven web 11 using glue or, preferably, hot-melt adhesive. The yarns may be attached to the web either in stretched condition or in relaxed condition, depending on the use of the nonwoven as the female loop fastener when making diapers or disposable absorbent briefs.

Figures 2A and 2B each illustrates other means of attachment of yarns to a substrate which form a loop fastener surface for the hook-and-loop fastener system of this invention. In Figure 2A the yarn, e.g., polyester yarn 413, is stitched or crocheted into the nonwoven substrate 415 and a non-tacky adhesive is sprayed thereon in order to enhance securement of the yarns to the nonwoven substrate 115. The adhesive is hot and tacky but becomes non-tacky after cooling, thus providing a non-tacky surface while insuring secure attachment of the yarns.

Figure 2B is similar to Figure 2A with the yarns 513 stitched or crocheted into the nonwoven surface 515. After spraying the adhesive as in Figure 2A, another nonwoven film 517 is adhered to the bottom surface of first nonwoven web to provide further securement and added structural integrity. The adhesive sprayed in this embodiment may be non-tacky, and may be the type of adhesives generally used in the construction of such absorbent articles.

The web used to provide the female loop fastening material may not only be any of a variety of known nonwoven materials, but may include a suitable woven fabric or even a thermoplastic film formed of polyolefin (e.g., polyethylene or polypropylene), polyester and other known thermoplastic materials. Nonwoven fabrics, however, are the substrate of choice because when used in making absorbent disposable briefs, they are skin-friendly and, in general, it is easier to attach the yarns onto the surface of a nonwoven fabric. For added structural integrity, the web 11 may be secured to a suitable backing of a thermoplastic polymer or a flexible film but such construction is not strictly required.

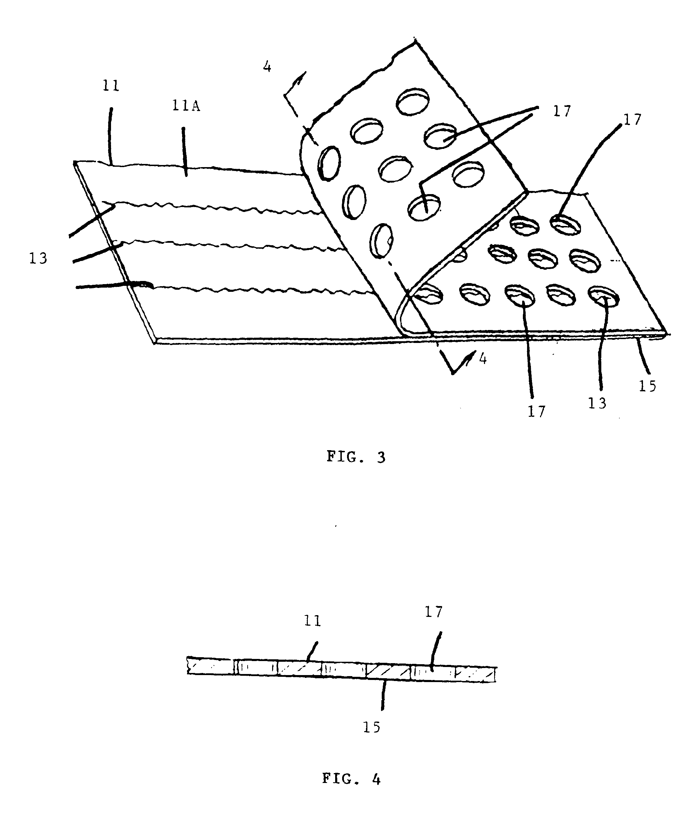

In another variation of the invention, the female loop fastener may be a composite web or laminate in the form illustrated in Figure 3. In accordance with this variation of the invention, the substrate or web 11, with the yarns 13 attached thereon as hereinbefore described, is covered by the superimposition thereon of an upper web 15 of nonwoven, woven tissue paper or thermoplastic film having a plurality of perforations 17, spaced apart and in rows coinciding with the rows of yarns which stretch across the web 11. The two webs or layers 11 and 15 may be adhesively secured to each other to form a composite, with the yarns exposed through the perforations 17 serving as the loop female fasteners adapted to be engaged or entangled by complementary mechanical hook components of a male fastener, such as, e.g., VELCRO® .

While four rows of the yarns 13 are shown stretched across the surface 11A of the web 11 in relatively straight parallel lines, fewer or greater number of rows of yarns may be used, and they may be stretched in other geometrical patterns such as undulated, zig-zag or V notches, and they may be stretched across the entire length of the web 11 or a part thereof, continuously or in a continual manner. Also, in lieu of merely adhesively attaching the yarns to the web, they may be sewn onto the surface 11A by the crotchet technique or by a combination of crotchet and adhesive securement as shown in figures 2A and 2B. It must be noted that the specific manner of attachment of the yarns to the web is not, per se, critical, so long as the yarns are adequately secured onto the surface of the web to provide engagement with complementary male hook elements. It must also be understood that the physical dimensions of the yarns and, in cases of twisted yarns, the numbers of twist per inch and other desired attributes of the yarns are well known in the art, and are described in prior art patents, including the aforementioned United States Patent No. 5,699,593.

As previously mentioned, either or both webs or layers which form the composite shown in Figure 3 may be replaced with a layer of a suitable thermoplastic material or even a woven web. If desired, the top layer or web 15 may be a gauze pad of suitable mesh, which is inherently flexible and sturdy, and is perforated thus exposing the yarns for engagement with complementary male hook fasteners.

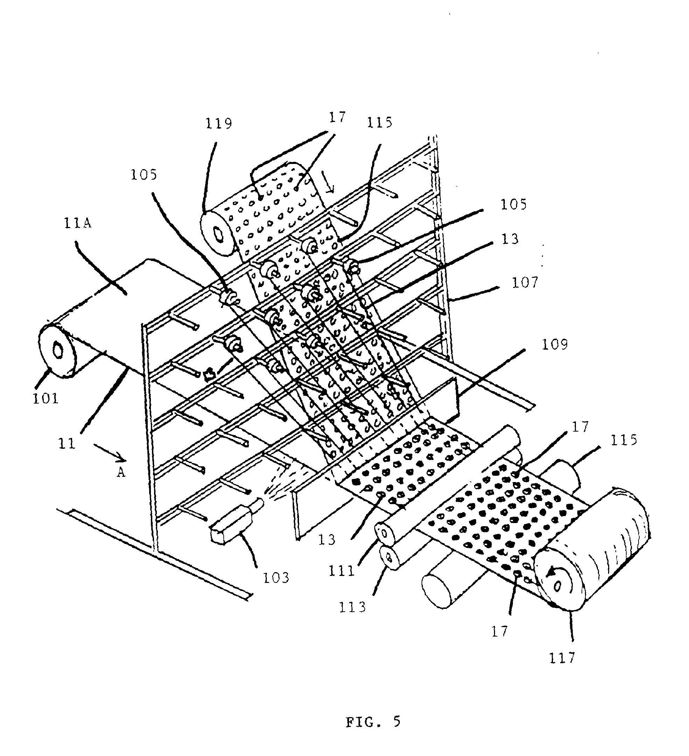

The method of attachment of the yarns to the surface of a suitable web to form a female loop fastener surface is illustrated in Figure 5. This figure also shows the method of producing a composite female loop fastener material. The method comprises, generally:
(a) feeding a web of nonwoven (layer or substrate) in a machine direction,
(b) spraying hot melt adhesive on the surface of the substrate, and
(c) simultaneously feeding the filamentary yarns in the same direction as the substrate direction, i.e., also in the machine direction, thereby causing the yarns to be adhesively secured to the surface of the nonwoven web.

In order to form a composite film, web or laminate as shown in Figure 3, the perforated cover web or film is fed in the machine direction simultaneously with feeding the base web and the two webs are glued together by the hot melt so as to form the composite web or laminate. Referring to Figure 5, the web of nonwoven fabric 11 is unrolled from the nonwoven roll 101 traveling in the direction of the arrow A, i.e., in the machine direction. The surface 11A of the web 11 is continuously sprayed with a hot melt adhesive from the adhesive vessel 103, and a plurality of filamentary yarns 13 are simultaneously unwound from respective set of creels or spools 105 which are supported in a structure such as the comb rack 107. The yarns 13 travel in the same direction as the web 11, i.e., in the machine direction, and are maintained spaced apart and under tension as they pass through the yarns grid 109 at which point the yarns adhere to the adhesive-bearing surface of the web 11. Naturally, the speed of travel of the web 11 and the yarns 13 are adjusted relative to each other, i.e., they are synchronized, so as to result in a web surface having a plurality of spaced apart yarns uniformly adhering thereon. Thereafter, the yarns 13 pass through the nip rolls 111 and 113 which may be chilled, or the yarn-bearing web may be passed through a separate chill roll 115 and wound or rolled on the windup roll 117.

If it is desired to form a composite web as described in connection with Figure 3, a separate but perforated web 15 (or a web of a thermoplastic film, a woven web of fabric or gauze) is unrolled from the roller 119, traveling in the same direction as the web 11. The webs 15 and 11 are adhered together by the adhesive hot melt which is sprayed on the web surface 11A. Again the speed of travel of the webs 11 and 15 are adjusted and synchronized so as to form the composite web in which the filamentary yarns 13 are visible through the perforations 17 with the yarns stretching preferably across the middle of each perforated area. These perforated areas define the sites for engagement of the male fastener elements onto the yarns.

Whether the female loop fastener of this invention is formed as a single layer (as in Figure 1) or as a composite (as in Figure 3), the invention provides a female loop surface adapted to engage or entangle complementary conventional male hooks such as, e.g., VELCRO® , or other male fastener elements.

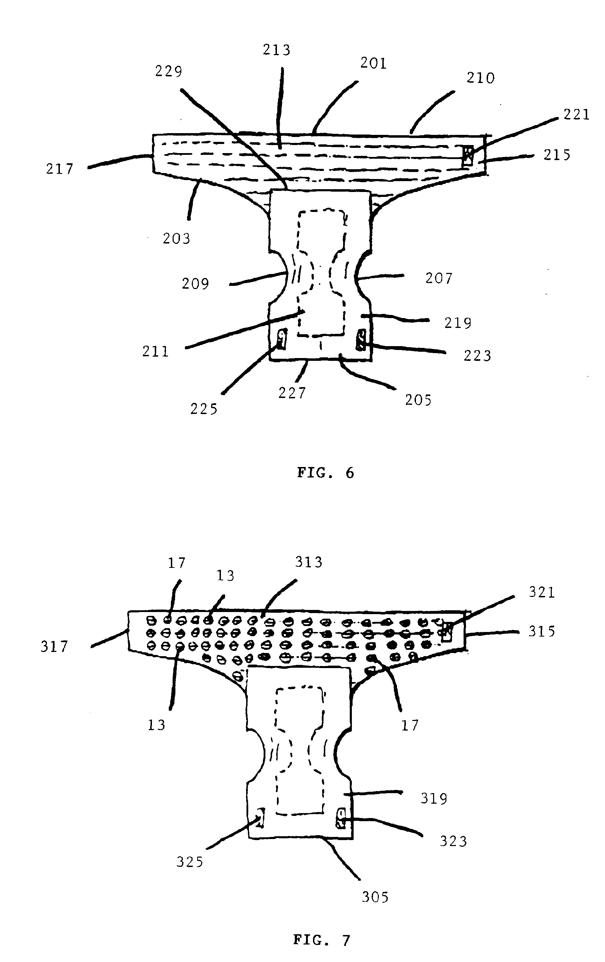

Figure 6 shows a T-shaped absorbent article (diaper) in plan stretched view, which incorporates a female loop fastening material of this invention. As shown in this figure, the diaper which is generally designated as 200 comprises a chassis having a back waist region 201, a front waist region 203, a crotch region 205 and a pair of leg openings 207 and 209. The diaper 200 also comprises a liquid pervious cover sheet and a liquid impervious backsheet having the usual conventional construction of T-shaped diapers such as the T-shaped diaper described in commonly assigned, copending application serial number 09/376,282 filed August 18, 1999, the disclosure of which is fully incorporated herein by reference.

Referring again to the T-shaped diaper shown in Figure 6, the back waist region 201 comprises the cross piece 213 which has a pair of laterally projecting end segments (ears) 215, 217, and a center piece or flap 219 having a distal end 227 and a proximal end 229 which extends vertically downward relative to the ears, 215, 217. In accordance with the present invention, the cross piece 213 of the diaper is formed partially or totally of female fastening material such as the single layer female loop fastening material shown in Figure 1, or a composite web made as in Figure 3, as hereinbefore described in this application. At least one VELCRO® male fastener 221 is provided on the portion 215 and a pair of spaced VELCRO® male fasteners 223, 225 are disposed at the distal end 227 of the center piece 219.

In order to wear the diaper 200, the ear 215 is folded toward the cross piece 213, i.e., around the waist of the wearer, followed by folding the ear 217 thereon causing the VELCRO® male fastener 221 to engage, the filamentary yarns 13 attached to the web which defines the cross piece 213. Thereafter, the center piece 219 is passed under the crotch region and folded over and upwardly onto the surface of the folded ear portions whereby the male VELCRO® male fasteners 223, 225 are engaged onto the female loop fastening material thus providing securement of the diaper around the waist of the wearer.

Figure 7 shows a diaper 300 which is similar in construction to Figure 6, the only difference being is that the cross piece 313 with its ears 315, 317 is defined by a composite (laminate) female loop fastener material made as in Figure 3. In this embodiment of the diaper, the VELCRO® male fasteners are so positioned that when the ear portions 315 and 317 are folded around the waist as aforesaid, the VELCRO® male fastener will be in register with one or more perforations 17 so as to result in entanglement of the VELCRO® male fastener with the filamentary yarn visible through the perforated area. Similarly, when the center piece 319 is passed under the crotch region 305 and as folded thereover as hereinbefore described, the VELCRO® male fasteners 323, 325 will be in register with complementary perforations 17 on the composite web and, accordingly, the VELCRO® male fasteners 323, 325 will become entangled with the filamentary yarns visible through the corresponding perforations with which they are in register, thus completing securement of the diaper around the waist of the wearer.

It is apparent from the foregoing description of the invention that several changes and modifications suggest themselves to those skilled in the art. Such changes relate, but are not limited to the material used to form the webs, yarn and yarn dimensions, size and shape of perforations when using composite laminate, the type of adhesive used to attach the yarns to the web, the relative spacings and arrangement of the several rows of yarns, etc.. All these changes and modifications are within the scope and contemplation of the present invention. Moreover, the loop fastener systems described herein may be useful and effective for other types of diapers, briefs and are not limited to the types of briefs and articles described herein.

## Claims

1. A loop fastening material suitable for engagement with a complementary male fastener element, said loop fastening material comprising a web having a top surface, at least one row of filamentary yarn secured to said top surface of said web, said yarns spanning substantially continuously along the length of said top surface, wherein said yarns are adapted to be entangled by complementary male fastener element.

2. A loop fastening material as in claim 1 wherein said web is made of a material selected from the group consisting of nonwoven fabric, woven fabric and thermoplastic polymer film.

3. A loop fastening material as in claim 2 wherein said thermoplastic polymer is selected from the group consisting of polyamide, polyolefin, polyester and mixtures thereof.

4. A loop fastening material as in claim 3 wherein said polyolefin is polypropylene.

5. A fastening material as in any of the claims 1-4 wherein said filamentary yarns are formed from a thermoplastic polymer.

6. A female fastening material suitable for engagement with a complementary male fastener element, said female fastening material being a composite web comprising a base web and a cover web, said base web having a top surface, at least one row of filamentary yarns secured to said top surface of said base web and spanning substantially along the length thereof, said cover web having a plurality of spaced apart perforations, said perforations being in a row and spaced apart so as to be in register with in the filamentary yarn on said lower web thereby defining exposed yarns sites through said perforations, said exposed yarns adapted to be entangled by complementary male fastener elements.

7. A female fastening material as in claim 6 wherein at least one of said webs is made of a material selected from the group consisting of nonwoven fabric, woven fabric and a thermoplastic polymer.

8. A female fastening material as in claim 7 wherein said thermoplastic polymer is selected from the group consisting of polyamide, polyolefin, polyester and mixtures thereof.

9. A female fastening material as in claim 8 wherein said polyolefin is polypropylene.

10. A female fastening material as in claim 6 wherein said perforated cover web is a gauze having at least 4 yarns per web in the machine direction and cross machine direction.

11. A loop fastening material suitable for engagement with a complementary male fastener element, said loop fastening material comprising a web having a top surface and a bottom surface, and at least one row of filamentary yarn stitched to said top surface, said yarn spanning substantially continuously along the length of said top surface, said bottom surface of said web comprising an adhesive for securement of said yarn, wherein said yarn is adapted to be entangled by complementary male fastener.

12. A loop fastening material as in claim 11 wherein said web is made of a material selected from the group consisting of nonwoven fabric, woven fabric and thermoplastic polymer film.

13. A loop fastening material as in claim 11 further including a second web adhesively secured to said bottom surface of said first web.

14. A loop fastening material as in claim 13 wherein each of said webs is made of a material selected from the group consisting of nonwoven fabric, woven fabric and thermoplastic material.

15. A method of making female fastening material which comprises:
(a) feeding a web of a material having a top surface, said web being selected from the group consisting of nonwoven fabric, woven fabric and a thermoplastic polymer film, said web traveling in one direction,
(b) applying adhesive to the top surface of said web as said web travels in said direction, thereby covering said top surface of the web with said adhesive,
(c) applying at least one row of filamentary yarn to said adhesive-covered surface of said web while said filamentary yarn is caused to travel in the same direction as the web, thereby producing a web with the filamentary yarn attached thereto.

16. A method as in claim 15 wherein a plurality of spaced apart yarns are caused to travel in the same direction as said web thereby producing a web having a plurality of spaced apart filamentary yarns attached to the top surface of said first web, in rows.

17. A method of making a composite female fastening material which comprises:
(a) feeding a first web of a material having a top surface, said web being selected from the group consisting of nonwoven fabric, woven fabric and a thermoplastic polymer film, said first web traveling in one direction,
(b) applying adhesive to the top surface of said first web as said web travels in said direction, thereby covering said top surface of said web with said adhesive,
(c) applying at least one row of filamentary yarn to said adhesive-covered surface of said web while said filamentary yarn is caused to travel in the same direction as the web, thereby producing a web with the filamentary yarn attached thereto, and
(d) feeding a second web of a material selected from the group consisting of nonwoven fabric, woven fabric, thermoplastic polymer film and gauze, said second web traveling in the same direction as the first web, said second web having at least one row of a plurality of spaced apart perforations,
whereby said second web is caused to adhere to said top surface of said first web to form a composite web with said perforations in register with the filamentary yarn on said first web thereby defining exposed sites of female fastening materials adapted to be entangled with complementary male fastening element.

18. A method as in claim 17 wherein a plurality of spaced apart yarns are caused to travel in the same direction as said first web thereby producing a web having a plurality of spaced apart filamentary yarns to the top surface of said first web, in rows.

19. An absorbent article having a chassis comprising two portions which together define a generally T-shaped configuration when said chassis is viewed in plan stretched position; a lateral portion, and a center portion having a distal end and a proximal end disposed generally vertically relative to said lateral portion and adapted to be passed under the crotch of a wearer and folded upwardly and over said first lateral portion;
said lateral portion having opposed lateral segments adapted to be wrapped around the torso of the wearer,
said lateral portion being defined by a loop fastening material having a surface and at least one row of filamentary yarns attached to said surface and spanning at least partly along said surface,
at least one male fastening means on one of said lateral segments,
a pair of opposed spaced apart male fastening means at the distal end of said center portion,
such that when said lateral segments are in overlapped position and said center portion is folded over said overlapped portions, said male fastening means engages said filamentary yarns on said surface of said lateral portion.

20. An absorbent article as in claim 19 wherein said lateral portion is shorter in length relative to said lateral portion.

21. An absorbent article as in claim 19 or 20 wherein said lateral portion is at least partly elasticated.

22. An absorbent article having a chassis comprising two portions which together define a generally T-shaped configuration when said chassis is viewed in plan stretched view; a lateral portion, and a center portion having a distal end and a proximal end disposed generally vertically relative to said lateral portion and adapted to be passed under the crotch of a wearer and folded upwardly and over said lateral portion;
said lateral portion having opposed lateral end segments adapted to be wrapped around the torso of the wearer,
at least one male fastening means on one of said lateral segments,
a pair of opposed spaced apart male fastening means at the distal end of said center portion,
said lateral portion being defined by a loop fastening composite material, said composite material having a base web and a cover web, said base web having a top surface, at least one row of filamentary yarn secured to said top surface of said base web and spanning substantially along the length thereof, said cover web having a plurality of spaced apart perforations, in rows, in register with said row of filamentary yarn and defining exposed yarns through said perforations,
wherein when said lateral end segments are in overlapped position, said male fastener on said lateral end segment engages exposed yarns on said overlapped portion, and
wherein when said center portion is folded over said overlapped portion of said lateral end segments, said male fasteners at the distal end of said center portion engage respective complementary yarns sites exposed through perforations in said cover web, thereby securing said absorbent articles around the torso of the wearer.

23. An absorbent article as in claim 22 wherein said lateral portion is shorter in length relative to said lateral portion.

24. An absorbent article as in claim 22 or 23 wherein said lateral portion is at least partly elasticated.
